# FASCICULE DE BREVET EUROPEEN

(11) **EP 1 246 642 B1**
(45) Date de publication et mention de la délivrance du brevet: **07.09.2005**
(21) Numéro de dépôt: 00993688.1
(22) Date de dépôt: 21.12.2000
(51) Int. Cl.: A61K 38/49, A61K 38/39, A61K 38/22, A61K 38/19, A61K 48/00, A61P 27/02

(54) **UTILISATION D'UN VECTEUR COMPRENANT UN ACIDE NUCLEIQUE CODANT POUR UN FACTEUR ANTI-ANGIOGENIQUE POUR LE TRAITEMENT DES NEOVASCULARISATIONS CORNEENNES**
VERWENDUNG VON EINEM FÜR EINEM ANTI-ANGIOGENEN FAKTOR CODIERENDEN VEKTOR ZUR BEHANDLUNG VON VASKULARISATION DER HORNHAUT
USE OF A VECTOR COMPRISING A NUCLEIC ACID CODING FOR AN ANTI-ANGIOGENIC FACTOR FOR TREATING CORNEAL NEOVASCULARIZATION

(30) Priorité: 30.12.1999 FR 9916780; 12.07.2000 US 217691 P
(43) Date de publication de la demande: 09.10.2002
(73) Titulaire: Aventis Pharma S.A., 92160 Antony (FR)
(72) Inventeur: ABITBOL, Marc, F-75013 Paris (FR)
(86) Numéro de dépôt international: PCT/FR2000/003653
(87) Numéro de publication internationale: WO 2001/049316

(56) Documents cités:
- WO-A-94/20146
- WO-A-99/29855
- US-A- 5 759 547
- US-A- 5 827 702
- DUEÑAS Z ET AL: "Inhibition of rat corneal angiogenesis by 16-kDa prolactin and by endogenous prolactin-like molecules" INVESTIGATIVE OPHTALMOLOGY & VISUAL SCIENCE (IOVS), vol. 40, no. 11, octobre 1999 (1999-10), pages 2498-2505, XP000952634

## Description

La présente invention concerne l'utilisation d'un vecteur comprenant un acide nucléique codant pour un facteur anti-angiogénique pour la prévention, l'amélioration et/ou le traitement des néovascularisations de la cornée. Elle concerne également des compositions pharmaceutiques et un dispositif permettant l'administration locale et efficace de ce vecteur.

Les kératopathies sont des pathologies de la cornée d'origine traumatique, chimique, infectieuse ou génétique. Les plus fréquentes sont d'origines infectieuses virales telles les kératopathies herpétiques ou zostériennes (zona ophtalmique, kératiques neuroparalytique). Les kératopathies traumatiques sont provoquées par des projections de petits objets tels les bris de pare-brise lors d'accidents de la circulation, et les kératopathies chimiques peuvent être dues à la projections de substances chimiques sur le globe oculaire.

L'ensemble de ces pathologies se complique souvent de néovascularisations qui sont en grande partie responsables de l'opacification de la cornée conduisant à la cécité. Le seul traitement actuellement disponible pour rendre la vue au patient atteint de kératopathie cécitante compliquée de néovascularisation est la greffe de cornée. Bien qu'efficace, cette approche est toutefois considérablement limitée par le manque de greffons : ainsi on observe en France un déficit annuel de 3000 greffons. Ce point illustre le besoin pour de nouveaux traitements des kératopathies cécitantes.

L'oeil est constitué d'un segment antérieur qui comprend la cornée, la chambre antérieure remplie d'humeur aqueuse, l'iris et le cristallin, et d'un segment postérieur dont font partie la rétine; la choroïde et la partie postérieure de la sclérotique. La cornée comprend, de la surface vers la profondeur de l'oeil, l'épithélium cornéen, la membrane basale de Bowman, le stroma cornéen et la membrane de Descernet sur laquelle repose l'endothélium cornéen. La cornée, enveloppe fibreuse et transparente, ne contient aucun vaisseau sanguin ni lymphatique. Elle est nourrie par diffusion des métabolites à partir de l'humeur aqueuse et des vaisseaux sanguins du limbe (jonction scléro-cornéenne), et reçoit une partie de l'O₂ directement à partir de l'environnement extérieur.

Le réseau sanguin de l'oeil dérive de l'artère ophtalmique par deux systèmes séparés, les vaisseaux rétiniens et le système vasculaire uvéal qui comprend les réseaux vasculaires de l'iris, du corps ciliaire et de la choroïde. Cet arbre vasculaire peut être modifié dans des circonstances pathologiques, caractérisées par une diminution du flux sanguin ou de la quantité d'oxygène. Des néovaisseaux prennent alors naissance à partir de petites boucles vasculaires issues de veinules, en bordure des zones d'ischémie. La membrane basale de ces veinules se fragmente sous l'effet d'enzymes protéolytiques et permet ainsi une migration des cellules endothéliales. Celles-ci changent de morphologie, se multiplient puis se creusent de vacuoles qui vont progressivement confluer et s'organiser pour former de véritables lumières vasculaires. La mauvaise étanchéité des jonctions interendothéliales et l'interruption de la membrane basale de ces néovaisseaux augmente leur perméabilité et entraîne une extravasation d'hématies.

Cette néovascularisation ou angiogénèse, est ainsi à l'origine de la plupart des maladies visuelles qui provoquent une baisse de l'acuité visuelle, voire la cécité. La prolifération des vaisseaux limbiques dans les couches superficielles de la cornée apparaît, dans de nombreux états pathologiques tels que certaines kératoconjonctivites virales , notamment celles causées par le virus de l'herpès, et les oedèmes. Une autre forme de néovascularisation susceptible d'entraîner la cécité se rencontre dans les cas de ptérygions dans lesquels la membrane d'origine conjonctivale avec des porte-vaisseaux progresse de la conjonctive péribulbaire vers la surface cornéenne, voir en profondeur vers le stroma cornéen. Les ptérygions peuvent également obliger à des greffes cornéennes parfois très difficiles et susceptibles à nouveau de provoquer des néovascularistions cornéennes.

Les inventeurs ont mis au point une méthode particulièrement efficace de prévention et/ou de traitement des néovascularisations cornéennes consistant dans l'administration au niveau de l'oeil d'une composition pharmaceutique comprenant un vecteur codant pour un facteur anti-angiogénique combinée à l'application d'une lentille de contact souple sur la cornée. La lentille de contact peut être appliquée préalablement, de manière concomitante ou postérieurement à l'administration de la composition comprenant le vecteur. De manière préférée, l'administration de la composition contenant le vecteur est concomitante à l'application de la lentille de contact et de préférence encore, l'administration se fait par imprégnation préalable de la lentille de contact avec la composition comprenant ledit vecteur avant l'application de.ladite lentille sur la cornée.

La composition pharmaceutique comprenant le vecteur peut être sous toute forme adaptée à un usage oculaire et notamment sous la forme de collyre ou de pommade ophtalmique. De manière préférée; la composition pharmaceutique se présente sous forme de collyre.

Pour étudier l'effet des facteurs anti-angiogéniques dans la prévention et le traitement des pathologies oculaires liées à la néovascularisation, la demanderesse a utilisé deux modèles animaux de rats présentant des néovascularisations cornéennes. Le transfert d'acides nucléiques codant pour des facteurs anti-angiogéniques a été réalisé au moyen d'adénovirus recombinants défectifs codant pour différents facteurs anti-angiogéniques administrés par instillation oculaire ou par application de lentilles souples préalablement imbibées de solution contenant des adénovirus. De manière inattendue, la demanderesse a mis en évidence que le transfert d'un vecteur comprenant un acide nucléique codant pour un facteur anti-angiogénique au niveau de la cornée au moyen d'une lentille de contact préalablement imprégnée d'une solution comprenant ledit vecteur permet de prévenir et de traiter les néovascularisations cornéennes avec une efficacité jamais égalée jusqu'alors.

Parmi les différents facteurs anti-angiogéniques utilisables dans le cadre de la présente invention, on peut citer notamment : le fragment N-terminal de l'activateur du plasminogène uPA (ATF) (Appella et al. J. Biol. Chem. 262, 4437-4440 (1987), l'angiostatine (M. O'Reilly *et al*. Cell 79, 1157-1164 (1994)), et en particulier l'angiostatine K3 (fragment N-terminal 1-333 du plasminogène humain), l'endostatine (M. O'Reilly *et al*. Cell 88, 277-285 (1997), le fragment 16 kDa de la prolactine (C. Clapp *et al*. Endocrinol. 133, 1292-1299 (1993)) ou le facteur plaquettaire 4 PF-4 (S.K. Gupta *et al*. P.N.A.S. USA 92, 7799-7803 (1995)).

Un premier objet de l'invention concerne l'utilisation d'un vecteur comprenant un acide nucléique codant pour au moins un facteur anti-angiogénique pour la préparation d'une composition pharmaceutique destinée à être administrée par imprégnation d'une lentille souple et application de ladite lentille sur la cornée pour la prévention, l'amélioration et/ou le traitement des néovascularisations cornéennes.

Préférentiellement, l'acide nucléique codant pour le facteur anti-angiogénique est un acide nucléique codant pour un polypeptide choisi parmi le fragment N-terminal de l'activateur du plasminogène uPA (ATF), l'angiostatine, l'angiostatine K3, l'endostatine, le fragment 16 kDa de la prolactine ou le facteur plaquettaire 4 (PF-4) ou une combinaison d'acides nucléiques codant pour au moins deux de ces facteurs. De manière préférée encore, le facteur anti-angiogénique est choisi parmi le fragment N-terminal de l'activateur du plasminogène uPA (ATF) et l'angiostatine K3.

Selon un mode particulier, le facteur anti-angiogénique est un variant des facteurs précédemment cités. Au sens de la présente invention on entend par "variant" d'un polypeptide ou d'une protéine tout analogue, fragment, dérivé ou forme mutée qui est dérivé d'un polypeptide ou d'une protéine et qui retient la fonction anti-angiogénique dudit polypeptide ou de ladite protéine. Différent variants d'un polypeptide ou d'une protéine peuvent exister à l'état naturel. Ces variants peuvent être des variations alléliques caractérisées par des différences dans la séquence nucléotidique des gènes de structure codant pour la protéine ou peuvent résulter d'un épissage différentiel ou de modifications post-traductionnelles. Ces variants peuvent être obtenus par substitution, délétion, addition et/ou modification d'un ou plusieurs résidus acides aminés. Ces modifications peuvent être réalisées par toutes techniques connues de l'homme du métier.

Ces variants sont notamment des molécules ayant une plus grande affinité pour leurs sites de fixation, des séquences permettant une expression améliorée in vivo, des molécules présentant une plus grande résistance aux protéases, des molécules ayant une efficacité thérapeutique plus grande ou des effets secondaires moindres, ou éventuellement de nouvelles propriétés biologiques.

D'autres variants utilisables dans le cadre de l'invention sont notamment les molécules dans lesquelles un ou plusieurs résidus ont été substitués, les dérivés obtenus par délétion de régions n'intervenant pas ou peu dans l'interaction avec les sites de liaison considérés ou exprimant une activité indésirable, et les dérivés confortant par rapport à la séquence native des résidus supplémentaires, tels que par exemple un signal de sécrétion ét/ou un peptide de jonction.

La séquence d'ADN codant pour le facteur anti-angiogénique utilisée dans le cadre de la présente invention peut être un ADNc, un ADN génornique (ADNg), ou une construction hybride consistant par exemple en un ADNc dans lequel seraient insérés un ou plusieurs introns. Il peut s'agir d'un acide nucléique d'origine animale ou humaine, de préférence il s'agit d'un acide nucléique d'origine humaine. Il peut également s'agir de séquences synthétiques ou semisynthétiques. De manière particulièrement avantageuse, on utilise un ADNc ou un ADNg. En particulier, l'utilisation d'un ADNg peut permettre une meilleure expression dans les cellules humaines.

Avantageusement, la séquence codant pour le facteur anti-angiogénique est placée sous le contrôle de signaux permettant son expression dans les cellules de l'épithélium cornéen. Préférentiellement, il s'agit de signaux d'expression hétérologues, c'est-à-dire de signaux différents de ceux naturellement responsables de l'expression du facteur anti-angiogénique. Il peut s'agir en particulier de séquences responsables de l'expression d'autres protéines, ou de séquences synthétiques. Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris l'adénovirus utilisé. A cet égard, on peut citer par exemple les promoteurs E1A, MLP, CMV, LTR-RSV, etc. En outre, ces séquences d'expression peuvent être modifiées par addition de séquences d'activation, de régulation, ou permettant une expression tissu-spécifique. Il peut en effet être particulièrement intéressant d'utiliser des signaux d'expression actifs spécifiquement ou majoritairement dans les cellules de la cornée, de manière à ce que la séquence d'ADN ne soit exprimée et ne produise son effet que lorsque le vecteur a effectivement infecté ces cellules.

L'acide nucléique codant pour un ou plusieurs facteurs anti-angiogéniques est introduit dans un vecteur. Au sens de la présente invention on entend par "vecteur" tout moyen permettant le transfert d'un acide nucléique dans une cellule hôte, de préférence au niveau des tissus de l'oeil et plus particulièrement au niveau de la cornée. Le terme vecteur comprend les vecteurs viraux et non-viraux pour le transfert d'un acide nucléique dans une cellule *in vivo* ou *ex vivo*. Un type de vecteur pour la mise en oeuvre de l'invention peut être par exemple un plasmide, un cosmide ou tout ADN non encapsidé par un virus, un phage, un chromosome artificiel, un virus recombinant, etc. Il s'agit de préférence d'un plasmide ou d'un virus recombinant.

Parmi les vecteurs de type plasmidique, on peut citer tous les plasmides de clonage et/ou d'expression connus de l'homme du métier et qui comportent généralement une origine de réplication. On peut citer également des plasmides portant des origines de réplication et/ou des marqueurs de sélection perfectionnés tels que décrits par exemple dans les demandes WO96/26270 et WO97/10343.

Parmi les vecteurs de type virus recombinant, on peut citer préférentiellement les virus adénovirus, rétrovirus, virus de l'herpès, les lentivirus, les virus adéno-associés recombinants ou le virus SV40. La construction de ce type de virus recombinants défectifs pour la réplication a été largement décrite dans la littérature, ainsi que les propriétés d'infection de ces vecteurs (voir notamment S. Baeck et K.L. March (1998), Circul. Research vol. 82, pp 295-305), T. Shenk, B.N. Fields, D.M. Knipe, P.M. Howley et al (1996), Adenoviridae : the viruses and their replication (in virology). Pp 211-2148, EDS - Ravenspublishers/Philadelphia, P. Yeh et M. Perricaudet (1997), FASEB Vol. 11, pp 615-623.

Un virus recombinant particulièrement préféré pour la mise en oeuvre de l'invention est un adénovirus recombinant défectif.

Les adénovirus sont des virus à ADN double brin linéaire d'une taille de 36 (kilobases) kb environ. Il en existe différents sérotypes, dont la structure et les propriétés varient quelque peu, mais qui présentent une organisation génétique comparable. Plus particulièrement, les adénovirus recombinants peuvent être d'origine humaine ou animale. Concernant les adénovirus d'origine humaine, on peut citer préférentiellement ceux classés dans le groupe C, en particulier les adénovirus de type 2 (Ad2), 5 (Ad5), 7 (Ad7) ou 12 (Ad12). Parmi les différents adénovirus d'origine animale, on peut citer préférentiellement les adénovirus d'origine canine, et notamment toutes les souches des adénovirus CAV2 [souche manhattan ou A26/61 (ATCC VR-800) par exemple]. D'autres adénovirus d'origine animale sont cités notamment dans la demande WO94/26914.

Le génome des adénovirus comprend notamment une séquence inversée répétée (ITR) à chaque extrémité, une séquence d'encapsidation (Psi), des gènes précoces et des gènes tardifs. Les principaux gènes précoces sont contenus dans les régions E1, E2, E3 et E4. Parmi ceux-ci, les gènes contenus dans la région E1 notamment sont nécessaires à la propagation virale. Les principaux gènes tardifs sont contenus dans les régions L1 à L5. Le génome de l'adénovirus Ad5 a été entièrement séquencé et est accessible sur base de données (voir notamment Genbank M73260). De même des parties, voire la totalité d'autres génomes adénoviraux (Ad2, Ad7, Ad12, etc) ont également été séquencées.

Pour leur utilisation comme vecteurs recombinants, différentes constructions dérivées des adénovirus ont été préparées, incorporant différents gènes thérapeutiques. Dans chacune de ces constructions, l'adénovirus a été modifié de manière à le rendre incapable de réplication dans la cellule infectée. Ainsi, les constructions décrites dans l'art antérieur sont des adénovirus délétés de la région E1, essentielle à la réplication virale, au niveau de laquelle sont insérées les séquences d'ADN hétérologue (Levrero et al., Gene 101 (1991) 195 ; Gosh-Choudhury et al., Gene 50 (1986) 161). Par ailleurs, pour améliorer les propriétés du vecteur, il a été proposé de créer d'autres délétions ou modifications dans le génome de l'adénovirus. Ainsi, une mutation ponctuelle thermosensible a été introduite dans le mutant ts 125, permettant d'inactiver la protéine de 72kDa de liaison à l'ADN (DBP) (Van der Vliet et al., J. Virol., 1975, 15(2) 348-354). D'autres vecteurs comprennent une délétion d'une autre région essentielle à la réplication et/ou à la propagation virale, la région E4. La région E4 est en effet impliquée dans la régulation de l'expression des gènes tardifs, dans la stabilité des ARN nucléaires tardifs, dans l'extinction de l'expression des protéines de la cellule hôte et dans l'efficacité de la réplication de l'ADN viral. Des vecteurs adénoviraux dans lesquels les régions E1 et E4 sont délétées possèdent donc un bruit de fond de transcription et une expression de gènes viraux très réduits. De tels vecteurs ont été décrits par exemple dans les demandes WO94/28152, WO95/02697, WO96/22378. En outre, des vecteurs portant une modification au niveau du gène IVa2 ont également été décrits (WO96/10088).

Dans un mode préféré de mise en oeuvre de l'invention, l'adénovirus recombinant est un adénovirus humain du groupe C. De manière plus préférentielle, il s'agit d'un adénovirus Ad2 ou Ad5.

Avantageusement, l'adénovirus recombinant utilisé dans le cadre de l'invention comprend une délétion dans la région E1 de son génome. Encore plus particulièrement, il comprend une délétion des régions E1a et E1b. A titre d'exemple, on peut citer des délétions affectant les nucléotides 454-3328; 386-3446 ou 357-4020 (par référence au génome de l'Ad5).

Selon une autre variante, l'adénovirus recombinant utilisé dans le cadre de l'invention comprend en outre une délétion dans la région E4 de son génome. Plus particulièrement, la délétion dans la région E4 affecte l'ensemble des phases ouvertes. On peut citer à titre d'exemple précis les délétions 33466-35535 ou 33093-35535. D'autres types de délétions dans la région E4 sont décrites dans les demandes WO95/02697 et WO96/22378.

La cassette d'expression contenant l'acide nucléique codant pour un facteur anti-angiogénique peut être insérée en différents sites du génome recombinant. Elle peut être insérée au niveau de la région E1, E3 ou E4, en remplacement des séquences délétées ou en surplus. Elle peut également être insérée en tout autre site, en dehors des séquences nécessaires en cis à la production des virus (séquences ITR et séquence d'encapsidation).

Au sens de la présente invention on entend par "cassette d'expression" d'un acide nucléique, un fragment d'ADN qui peut être inséré dans un vecteur à des sites de restrictions spécifiques ; le fragment d'ADN comprend, outre la séquence nucléotidique codant pour un ARN ou un polypeptide d'intérêt, les séquences nécessaires à l'expression (enhanceur(s), promoteur(s), séquence de polyadénylation ...) de ladite séquence d'intérêt. Le fragment d'ADN et les sites de restriction sont conçus pour assurer une insertion dudit fragment dans un cadre de lecture approprié pour la transcription et/ou la traduction.

Les adénovirus recombinants sont produits dans une lignée d'encapsidation, c'est-à-dire une lignée de cellules capables de complémenter en trans une ou plusieurs des fonctions déficientes dans le génome adénoviral recombinant. Parmi les lignées d'encapsidation connues par l'homme du métier, on peut citer par exemple la lignée 293 dans laquelle une partie du génome de l'adénovirus a été intégrée. Plus précisément, la lignée 293 est une lignée de cellules embryonnaires humaines de rein contenant l'extrémité gauche (environ 11-12 %) du génome de l'adénovirus sérotype 5 (Ad5), comprenant 1TTR gauche, la région d'encapsidation, la région E1, incluant E1a et E1b, la région codant pour la protéine pIX et une partie de la région codant pour la protéine pIVa2. Cette lignée est capable de trans-complémenter des adénovirus recombinants défectifs pour la région E1, c'est-à-dire dépourvus de tout ou partie de la région E1, et de produire des stocks viraux ayant des titres élevés. Cette lignée est également capable de produire, à température permissive (32°C), des stocks de virus comportant en outre la mutation E2 thermosensible. D'autres lignées cellulaires capables de complémenter la région E1 ont été décrites, basées notamment sur des cellules de carcinome de poumon humain A549 (WO94/28152) ou sur des rétinoblastes humains (Hum. Gen. Ther. (1996) 215). Par ailleurs, des lignées capables de trans-complémenter plusieurs fonctions de l'adénovirus ont également été décrites. En particulier, on peut citer des lignées complémentant les régions E1 et E4 (Yeh et al., J. Virol. Vol. 70 (1996) pp 559-565; Cancer Gen. Ther. 2 (1995) 322 ; Krougliak et al., Hum. Gen. Ther. 6 (1995) 1575) et des lignées complémentant les régions E1 et E2 (WO94/28152, WO95/02697, WO95/27071).

Les adénovirus recombinants sont habituellement produits par introduction de l'ADN viral dans la lignée d'encapsidation, suivie d'une lyse des cellules après environ 2 ou 3 jours (la cinétique du cycle adénoviral étant de 24 à 36 heures). Pour la mise en oeuvre du procédé, l'ADN viral introduit peut être le génome viral recombinant complet, éventuellement construit dans une bacterie (WO96/25506) ou dans une levure (WO95/03400), transfecté dans les cellules. Il peut également s'agir d'un virus recombinant utilisé pour infecter la lignée d'encapsidation. L'ADN viral peut aussi être introduit sous forme de fragments portant chacun une partie du génome viral recombinant et une zone d'homologie permettant, après introduction dans la cellule d'encapsidation, de reconstituer le génome viral recombinant par recombinaison homologue entre les différents fragments.

Après la lyse des cellules, les particules virales recombinantes sont isolées par centrifugation en gradient de chlorure de césium. Une méthode alternative a été décrite dans la demande WO98/00528 incorporée à la présente par référence.

A titre d'exemple de vecteur convenant pour la mise en oeuvre de la présente invention on peut citer notamment : l'adénovirus recombinant défectif comprenant le gène codant pour le fragment ATF (Ad.CMV.ATF) tel que décrit dans la demande WO98/49321, l'adénovirus recombinant défectif comprenant le gène codant l'angiostatine K3 (Ad-K3) tel que décrit dans la demande WO98/49321.

L'invention concerne également une composition pharmaceutique comprenant un vecteur tel que décrit ci-avant et un véhicule physiologiquement acceptable pour une formulation destinée à être administrée dans l'oeil, en particulier par instillation. Il peut s'agir en particulier de solutions salines (phosphate monosodique, disodique, chlorure de sodium, potassium, calcium ou magnésium, etc, où des mélanges de tels sels), stériles, isotoniques, ou de compositions sèches, notamment lyophilisées, qui, par addition selon le cas d'eau stérilisée ou de sérum physiologique, permettent la constitution de solutés destinés à une instillation oculaire.

Les doses utilisées pour l'instillation ou l'imprégnation des lentilles de contact peuvent être adaptées en fonction de différents paramètres, et notamment en fonction du mode d'administration utilisé, de la nature chimique des lentilles de contact, du gène à exprimer, ou encore de la durée de l'expression recherchée. D'une manière générale, les virus recombinants selon l'invention sont formulés et administrés sous forme de doses comprises entre 10⁴ et 10¹⁴ pfu, et de préférence 10⁶ à 10¹⁰ pfu. Le terme pfu ("plaque forming unit") correspond au pouvoir infectieux d'une solution virale, et est déterminé par infection d'une culture cellulaire appropriée, et mesure du nombre de plages de cellules infectées. Les techniques de détermination du titre pfu d'une solution virale sont bien documentées dans la littérature.

En outre, les composition selon l'invention peuvent également comprendre un agent de transfert chimique ou biochimique. On entend par le terme "agent de transfert chimique ou biochimique " tout composé (i.e., autre qu'un virus recombinant) facilitant la pénétration d'un acide nucléique dans une cellule. Il peut s'agir d'agents non viraux cationiques comme des lipides cationiques, des peptides, des polymères (Polyéthylène Imine, Polylysine), des nanoparticules ; ou d'agents non viraux non cationiques comme des liposomes non cationiques, des polymères ou des nanoparticules non cationiques.

Selon un mode préféré, les compositions selon l'invention comprennent un vecteur recombinant défectif comprenant un gène codant pour un facteur anti-angiogénique et sont formulées pour une instillation intraoculaire. Avantageusement, les compositions de l'invention comprennent de 10⁴ à 10¹⁴ pfu, et de préférence de 10⁶ à 10¹⁰ pfu. Le titre des solutions utilisées pour l'imprégnation des lentilles de contact est compris entre 1. 10⁶ et 1. 10¹² pfu / ml et de préférence entre 1. 10⁸ et 1. 10¹⁰ pfu / ml.

L'invention concerne également un kit comprenant un récipient contenant une composition telle que précitée et au moins une lentille de contact et l'utilisation du kit pour la préparation d'un médicament destiné à être administré par imprégnation d'une lentille pour la prévention, l'amélioration et/ou le traitement des néovascularisations cornéennes.

Les lentilles de contact utilisable dans le cadre de la présente invention sont bien connues de l'homme du métier et sont de préférence des lentilles de contact souples, telles que décrites notamment dans Künzler *et al*. (*Chemistry* & *Industry*, 651-655 (1995) ; Künzler *et al*. (TRIP, Vol 4 (2) 52-59 (1996) ; J. Singh *et al.* (*J.M.S. Rev. Macromol. Chem. Phys.* C32(3&4), 521-534 (1992)) ; J.C. Wheeler et al. (*Journal of Long-Term Effects of Medical Implants*, 6 (3&4) : 207-217 (1996)).

L'invention a également pour objet un procédé de préparation d'un médicament utile pour la prévention, l'amélioration et/ou le traitement des néovascularisations cornéennes caractérisé en ce que l'on mélange un vecteur recombinant comprenant un acide nucléique codant pour un facteur anti-angiogénique avec un ou plusieurs adjuvants compatibles et pharmaceutiquement acceptables.

La présente invention sera décrite plus en détail à l'aide des exemples qui suivent qui doivent être considérés comme illustratifs et non limitatifs.

### LEGENDE DES FIGURES

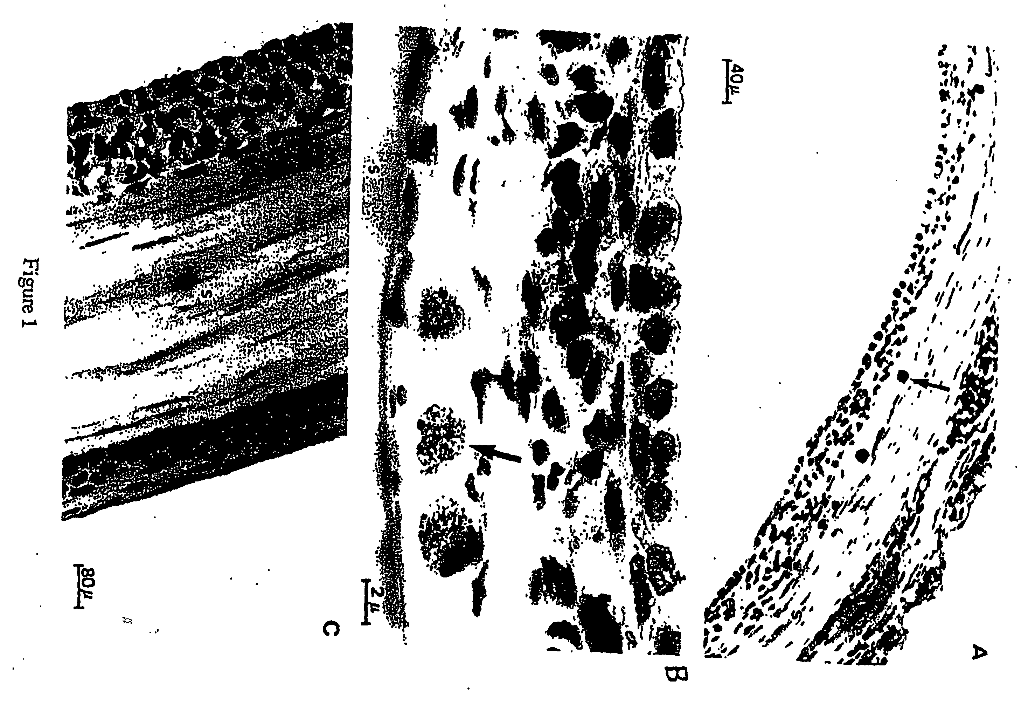

Figure 1 : Révélation de l'expression de la β-galactosidase par immunohistochimie sur coupe de cornée de rat adulte, incluse dans de la paraffine (5 µm) et ayant été en contact avec une lentille imprégnée d'Ad. β-gal 2. 10⁷ pfu), durant 72. h. Coloration à l'Hémalun. (A) Cornée néovascularisée suite à un grattage superficiel, grossissement x 265. (B) marquage nucléaire de cellules épithélialesrévélées avec un anticorps antiβ-gal, grossissement x 1310. (C) Cornée témoin normale avasculaire, grossissement x 525. (e : épithélium ; en: endothélium ; néovx : néovaisseaux ; s : stroma).

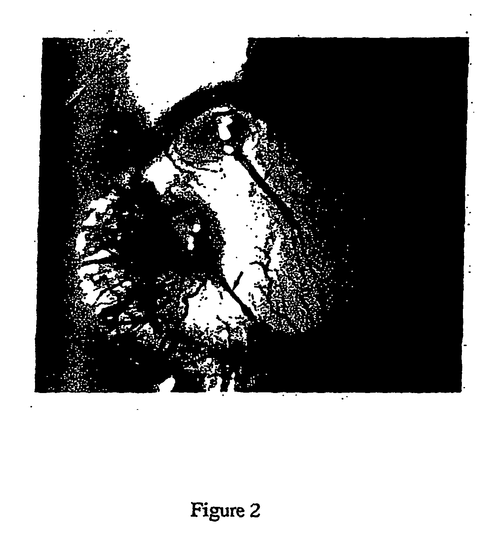

Figure 2 : photographie d'une néovascularisation cornéenne (triangle noir) induite par sutures (flèches noires) placées dans la cornée d'un rat adulte deux semaines plus tôt. Grossissement x 24.

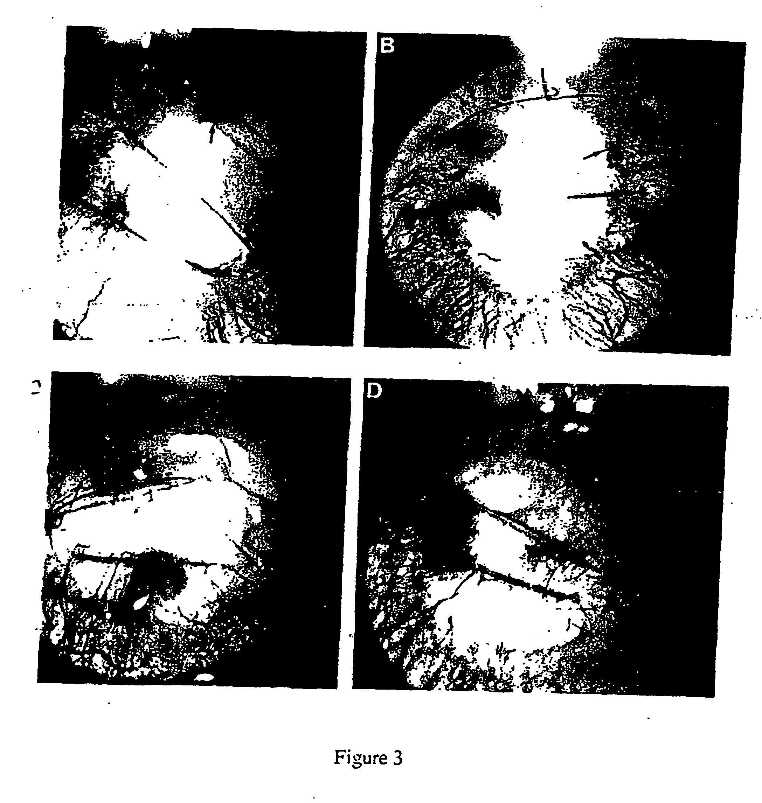

Figure 3 : Effet anti-angiogénique préventif d'une lentille impégnée d'une solution adénovirale (Ad.CMV.ATF, figure (B) et (D) ; AdK3 , figure (A) et (C)). Grossissement x 20. (A) et (B) correspondent à des cornées qui sont restées en contactde la lentille pendant une semaine. (C) et (D) correspondent à des cornées qui sont restées en contact de la lentille pendant deux semaines.

### MATERIEL ET METHODES

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extractions de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondamment décrites dans la littérature [Maniatis T. et al., "Molecular Cloning, a Laboratory Manual", Cold Spring Harbor Laboratory, Cold Spring Harbor, N,Y., 1982; Ausubel F.M. et al. (eds), "Current Protocols in Molecular Biology", John Wiley & Sons, New York, 1987.

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Modèle de néovascularisation cornéenne chez le rat

Trente rats Wistar mâles adultes, de 150 g, sont anesthésiés par une injection intrapéritonéale de 15 mg de Kétamine et 1.5 mg de Xylazine. L'anesthésie générale est complétée par une anesthésie topique à l'aide d'une goutte de chlorydrate d'oxybuprocaïne (Novésine®) au niveau cornéen. Deux modèles ont été testés : un modèle par suture interrompues et un modèle par grattage.

Modèle par sutures interrompues : vingt rats sont utilisés pour évaluer ce mode d'induction de néovascularisation cornéenne. Trois sutures interrompues (Nylon 8-0, aiguille 50 µm) sont placées à travers la cornée centrale de l'oeil droit de l'animal dans des conditions aseptiques, en utilisant un microscope opératoire (Zeiss). L'oeil gauche non opéré sert de témoin. Les cornées sont suivies avec un biomicroscope photographique pour le développement des néovaisseaux cornéens. Pour quantifier la néovascularisation cornéenne ,en double aveugle, l'oeil est divisé en quatre quadrants cornéens identiques et la néovascularisation de chacun est graduée de 0 à 3, par tranches de 0.5. Ainsi, le score pour chaque oeil s'étale de 0 à 12 (W. Streilen et al. 1996, Invest. Ophtamol. Vis. Sci. 37 : 413-424).

Modèle par grattage : les épithéliums cornéen et limbique de l'oeil droit de dix rats sont retirés par grattage chirurgical pendant 10 min. à l'aide d'un scalpel 15°, puis par débridement à l'éthanol (EtOH) 100° sous un microscope opératoire (Zeiss), ( A. HUANG *et al.* 1988, Ophtalmology 95, 228-235). Les cornées dénudées cicatrisent en une dizaine de jours et se vascularisent. La néovascularisation cornéenne est ensuite évaluée comme décrit dans le modèle par sutures interrompues, l'oeil gauche non opéré servant de témoin.

Préparation et pose des lentilles : des lentilles souples jetables sont taillées avec un emporte-pièce d'un diamètre inférieur à celui de la cornée du rat (3 mm) puis sont mises en présence, pendant une à deux heure(s) à température ambiante, des solutions adénovirales AdK3, Ad.CMV.ATF ou Ad.CMVβgal, respectivement de 4.5x10⁵ pfu (plaque forming unit), 10⁶ pfu et 2x10⁷ pfu pour chaque lentille. Des lentilles témoins sont également réalisées avec du glycérol à 10%, véhicule dans lequel chaque adénovirus est dilué. Une fois la lentille mise en place sur la cornée droite du rat, l'oeil est soigneusement refermé en pratiquant une tarsorraphie (soie 6-0, aiguille 50 µm), l'oeil gauche non opéré servant de témoin.

L'expression du transgène par les adénovirus est détectée grâce aux techniques (1) de révélation par colorimétrie sur coupes congelées, pour la révélation de la β-galactosidase uniquement, ou (2) d'immunohistochimie sur coupes paraffine.
(1) Des rats Wistar nouveaux-nés reçoivent une lentille d'Ad.CMVβgal sur l'oeil droit Pour cela, les animaux sont anesthésiés par immersion dans la glace puis la paupière est soigneusement coupée parallèlement à sa future ligne d'ouverture, à l'aide de ciseaux de Dowell coudés à 45° et d'une pince microchirurgicale. Une fois la lentille placée sur la cornée, l'oeil est refermé à l'aide de soie 6.0.
(2) Douze rats adultes sans néovascularisation cornéenne (quatre animaux par lot) reçoivent une lentille soit d'AdK3, soit d'Ad.CMV.ATF ou d'Ad.CMVβgal sur l'oeil droit, et 3 rats avec néovascularisation sont opérés avec une lentille d'Ad.CMVβgal. Une tarsorraphie est réalisée pour refermer l'oeil opéré.

Techniques de révélation : trois techniques différentes sont utilisées pour détecter la β-galactosidase. Les animaux sont sacrifiés par élongation cervicale 72 h après la pose de la lentille, les paupières sont ouvertes et les yeux énucléés à la pince. La β-galactosidase est détectée sur oeil entier ou sur des coupes congelées de 14 µm ou sur des coupes paraffine de 5 µm d'épaisseur.
(i) Sur oeil entier : après fixation dans du glutaraldéhyde 0.5 % pendant 1 h à température ambiante, chaque oeil (de rat adulte ou nouveau-né) est plongé dans une solution de PBS 1X contenant 2 mM de MgCl₂ puis est débarrassé des tissus musculaires résiduels sous loupe binoculaire (Leica). Chaque oeil est alors incubé dans une solution de révélation de 5 mM K3Fe(CN)₆, 5 mM K4Fe(CN)₆, 2 mM MgCl₂, 1 mg/ml 5-bromo-4-chloro-3-indolyl-b-D galactoside (X-gal) pendant 2 h, à 37°C. Après plusieurs lavages dans du PBS 1x, l'oeil est photographié sous loupe binoculaire.
(ii) Coupes congelées de 14 µm : les yeux de rat nouveau-né sont immédiatement fixés dans du glutaraldéhyde 0.5 % pendant 1 h à température ambiante puis sont cryoprotégés dans du sucrose 15 % pendant 30 min. Les yeux sont ensuite déposés dans des cupules en plastique solide contenant un milieu d'inclusion (Tissue Tek) qui, une fois congelé dans de l'isopentane à -30°C, permet de conserver les tissus à -80°C. Les yeux sont coupés à 14 µm au cryostat (Leica). La solution de révélation est ensuite déposée directement sur les coupes, en chambre humide, pendant 2 h à 37°C. Après trois rinçages de 10 min. en PBS lx, les coupes sont contre-colorées au rouge neutre 1 %, déshydratées dans des bains de degré d'alcool croissant : 80°, 95° et 100° puis montées entre lame et lamelle dans de l'eukitt.
(iii) Coupes paraffine de 5 µm d'épaisseur : la méthode de détection de la β-galactosidase par immunohistochimie est une technique beaucoup plus sensible que la méthode de révélation classique par colorimétrie. Après 48 heures d'immersion dans le fixateur de Davidson, les yeux de rat adulte sont disséqués soigneusement afin de retirer le cristallin, disposés dans une cassette plastique et placés dans le panier d'un automate qui réalise le cycle de traitement suivant : Formol 10%, 30 min. ; EtOH 70°, 1 h ; EtOH 80°, 1 h ; EtOH 100°, 5 fois 1 h ; xylène, 2 fois 1h30 ; paraffine, 4 fois 1 h. Les globes sont ensuite inclus dans la paraffine et coupés en sections de 5 µm grâce au microtome (Leica), de façon sagittale, parallèlement au nerf optique. Les coupes sont recueillies sur des lames pré-traitées (DAKO).

Fixation des coupes : pour bénéficier d'une révélation immunohistechimique, avec un anticorps anti β-galactosidase (TEBU), les lames sont séchées en étuve à 56°C pendant 48 h puis déparaffinées dans 2 bains de xylène de 15 min. et 2 bains d'EtOH 100° de 10 min. Elles sont ensuite réhydratées quelques minutes dans l'eau courante.

Bloquage et perméabilisation : les lames sont placées dans du tampon citrate 0.01 M ; pH 6, d'abord à température ambiante pendant 5 min., puis dans un four à micro-ondes à puissance 8 (750 Watts), 2 fois 5 min. Revenues à température ambiante, les coupes sont rincées dans de l'eau osmosée et cerclées au DAKO pen. Afin d'éliminer les péroxydases tissulaires qui peuvent interférer avec le complexe de révélation et rendre ainsi la réaction ininterprétable, nous utilisons de la H₂O₂ 3 % pendant 10 min. Les lames sont ensuite préincubées pendant 30 min. dans un mélange PBS 1x, gélatine 2 g/l, triton 0.25 % et albumine de sérum bovine (BSA) à 3 % pour saturer les sites aspécifiques.

Marquage : l'anticorps primaire de lapin anti β-galactosidase est dilué au 1/1000 dans un mélange de PBS 1x, gélatine 2 g/l et triton 0.025%. Après 1 h d'incubation à température ambiante, un brefrinçage puis quatre autres de 5 min. sont réalisés avec le mélange ayant servi à diluer l'anticorps. L'anticorps secondaire biotinylé anti-lapin, produit chez l'âne (DAKO), est dilué au 1/200 dans du PBS lx additionné de gélatine à 2 g/l, et appliqué pendant 30 min. Les lames sont ensuite rincées, d'abord rapidement, puis 4 fois 5 min., avec le milieu de dilution avant d'ajouter le complexe de révélation streptavidine/péroxydase (DAKO) dilué au 1/400 dans le même mélange, pendant 30 min. Après trois lavages de 5 min., le substrat chromogène, la diammobenzidine (DAB), est ajouté. Le temps de révélation varie de 2 à 20 min. La réaction est stoppée en plongeant les lames dans de l'eau osmosée. Celles-ci sont ensuite contre-colorées dans de l'Hémalun pendant 1 min., rincées rapidement dans de l'eau osmosée puis différenciées dans du LiCO₃ quelques secondes. Elles sont finalement déshydratées puis conservées dans du xylène jusqu'au montage entre lame et lamelle dans de l'eukitt.

### Exemple 1 : Construction des adénovirus recombinants défectifs Ad.CMV.ATF , AdK3 et Ad.CMVβgal.

Les vecteurs ont été construits selon la méthode générale décrite par Crouzet et al (PNAS vol 94 p1414, 1997). Le détail de la construction des secteurs Ad.CMV.ATF et AdK3 a été décrit dans la demande WO98/49321.

Pour l'AdK3, le transgène de 1 Kb correspond au fragment N-terminal du plasminogène humain (jusqu'au résidu 333) et inclut les 3 premiers domaines kringles de la molécule d'angiostatine.

Pour l'Ad.CMV.ATF, le transgène de 0.5 Kb est un ADNc codant pour le fragment N-terminal de l'uPA murin (acides aminés 1 à 135) et le transgène de l'Ad.CMVβgal comprend un ADNc de 3.1, Kb codant pour le gène rapporteur d'*Escherichia coli, lacZ*.

La production et la sécrétion de molécules adénovirales à partir des constructions ont été respectivement vérifiées par Northern-blot et Western-blot. Des tests d'inhibition *in vivo*, de la croissance tumorale, de l'angiogénèse et de la tumorigénèse, ainsi que des tests d'inhibition *in vitro,* de la prolifération cellulaire stimulée par le bFGF ont été réalisées pour les AdK3 et Ad.CMV.ATF. L'activité X-gal de l'Ad.CMVβgal a également été contrôlée.

Le titre moyen des solutions stock des adénovirus recombinants utilisés est de 4.5x10⁸ pfu/ml pour l'AdK3, 10⁹ pfu/ml pour l'Ad.CMV.ATF et 6.9x10¹⁰ pfu/ml pour l'Ad.CMVβgal.

### Exemple 2 : effet d'un vecteur codant pour un facteur anti-angiogénique dans un modèle de néovascularisation cornéenne chez le rat

L'effet de la lentille sur l'oeil droit de l'animal est étudié en fonction (1) de la durée du contact avec la cornée -une ou deux semaine(s)-, (2) de la présence ou non du vecteur codant pour un facteur anti-angiogénique - (3) de la néovascularisation ou non de la cornée.

Pour rechercher un effet thérapeutique "préventif" de l'adénovirus sur la néovascularisation cornéenne, la lentille est placée sur la cornée immédiatement après la pose des 3 sutures interrompues, tandis qu'un effet "curatif" est étudié en posant la lentille sur des yeux présentant déjà une néovascularisation cornéenne, depuis une ou deux semaine(s).

Chaque adénovirus est testé sur un lot de 10 rats : 5 pour évaluer l'effet préventif et 5 pour l'effet curatif. Un lot de 10 rats avec une lentille imprégnée de véhicule sert de témoin.

### Evaluation des deux modèles animaux de néovascularisation cornéenne

Le modèle d'induction de néovaisseaux cornéens par la pose de trois sutures interrompues est très efficace puisque 100% des animaux opérés présentent une néovascularisation cornéenne, et la cornée des yeux contrôles contralatéraux est avasculaire. Les vaisseaux sont attirés vers le centre de la cornée, à partir des arcades vasculaires limbiques et prolifèrent dans les couches superficielles de la cornée dès la première semaine suivant l'intervention.

Le score moyen de chaque oeil, indiquant le développement de la néovascularisation cornéenne, est de 7 ± 2, deux semaines après la pose des sutures. A ce moment-là, la néovascularisation cornéenne est maximale. Dans le modèle d'induction par grattage superficiel de la cornée seulement 70% des rats développent une néovascularisation cornéenne. Toutefois, celle-ci est plus homogène que celle du modèle précédent car l'ensemble des quatre quadrants de l'oeil participe au développement de la néovascularisation contrairement à ce que l'on observe dans le modèle des sutures où le nombre de quadrants impliqués est variable. Cette variabilité de l'étendue de la surface cornéenne néovascularisée est étroitement dépendante de la position des sutures par rapport au limbe, de leur profondeur dans le stroma et de leur taille. Le score moyen de chaque d'oeil est de 8 ± 2, deux semaines après grattage.

Sur une coupe de cornée incluse dans la paraffine, colorée avec un mélange d'acide périodique/base de Schiff/hématoxyline, les néovaisseaux apparaîssent de façon prédominante dans l'épithélium cornéen, mais également dans le stroma et au contact de l'endothélium cornéen.

### Efficacité du transfert des vecteurs adénoviraux par imprégnation préalable d'une lentille de contact

Résultats macroscopiques : après incubation avec la solution X-gal, les cupules oculaires de tous les yeux ayant été exposés à une lentille imprégnée avec de l'adénovirus β-galactosidase à 2 x 10⁷ pfu/µl présentent une coloration bleue ponctuée et très diffuse sur toute la cornée du rat nouveau-né et sur une large région de la cornée des animaux adultes. La coloration bleue n'est retrouvée sur aucun des yeux témoins.

Résultats microscopiques : le marquage obtenu par révélation colorimétrique de la β-galactosidase sur coupes congelées de cornées normales prédomine dans le noyau des cellules épithéliales cornéennes.

Sur coupes de cornées néovascularisées incluses dans la paraffine, le marquage obtenu par immunohistochimie est principalement retrouvé dans les noyaux des cellules endothéliales cornéennes, mais aussi dans celui des cellules épithéliales cornéennes et épithéliales des corps ciliaires. Les kératocytes du stroma cornéen ainsi que des cellules endothéliales intrastromales pathologiques sont également marquées (Figure 1). Malgré le signal de localisation nucléaire adjoint au transgène lacZ, certaines cellules présentent un marquage dans le cytoplasme, quelle que soit la technique de révélation.

Des résultats identiques sont obtenus à l'aide d'adénovirus codant pour l'ATF murin ou pour l'angiostatine humaine. Cela a pu être démontré par des réactions immunohistochimiques spécifiques, réalisées sur coupes de globes oculaires de rat inclus dans la paraffine.

### Effet anti-angiogénique des lentilles préalablement imprégnées d'adénovirus Ad.CMV.ATF et AdK3.

Des lentilles imprégnées de véhicule et placées sur une cornée sans néovascularisation n'ont aucun effet angiogénique significatif.

Aucun phénomène inflammatoire et aucune néovascularisation cornéenne ne sont induits par les lentilles imbibées d'Ad.CMVβgal à 2 x 10⁷ pfu/µl

De même, les lentilles d'Ad.CMV.ATF (10⁶ pfu/µl) et d'AdK3 (4.5 x 10⁵ pfu/µl) n'induisent aucune réaction inflammatoire sur la cornée, aucune néovascularisation, ni de disparition de la vascularisation limbique.

Une lentille imbibée d'Ad.CMV.ATF placée sur une cornée immédiatement après la pose des trois sutures interrompues, prévient le développement de la néovascularisation attendue suite à la mise en place de ces trois sutures, pendant la première semaine. Cet effet persiste après deux semaines. On observe le même effet anti-angiogénique au bout d'une semaine de contact avec la cornée, avec une lentille imbibée d'AdK3 (Figure 3)

## Revendications

1. Utilisation d'un vecteur comprenant un acide nucléique codant pour un facteur anti-angiogénique pour la préparation d'une composition pharmaceutique destinée à être administrée par imprégnation d'une lentille souple et application de ladite lentille sur la cornée pour la prévention, l'amélioration et/ou le traitement des néovascularisations cornéennes.

2. Utilisation selon la revendication 1, **caractérisée en ce que** l'acide nucléique codant pour le facteur anti-angiogénique est un acide nucléique codant pour un polypeptide choisi parmi le fragment N-terminal de l'activateur du plasminogène uPA (ATF), l'angiostatine, l'endostatine, le fragment 16 kDa de la prolactine ou le facteur plaquettaire 4 (PF-4) ou une combinaison d'acides nucléiques codant pour au moins deux de ces facteurs.

3. Utilisation selon la revendication 2, **caractérisée en ce que** le facteur anti-angiogénique est choisi parmi le fragment N-terminal de l'activateur du plasminogène uPA (ATF) et l'angiostatine K3.

4. Utilisation selon l'une des revendications 1 à 3, **caractérisée en ce que** le vecteur est un plasmide, un cosmide ou tout ADN non encapsidé par un virus.

5. Utilisation selon l'une des revendications 1 à 3 **caractérisée en ce que** le vecteur est un virus recombinant, de préférence dérivé d'un adénovirus, d'un rétrovirus, d'un lentivirus, d'un virus de l'herpès ou d'un virus adéno-associé.

6. Utilisation selon la revendication 5, **caractérisée en ce que** le virus recombinant est un adénovirus recombinant défectif.

7. Utilisation selon la revendication 5 ou 6, **caractérisée en ce que** la composition pharmaceutique comprend entre 1. 10⁶ et 1. 10¹² pfu / ml et de préférence entre 1. 10⁸ et 1. 10¹⁰ pfu / ml.

8. Procédé de préparation d'un médicament utile pour la prévention, l'amélioration et/ou le traitement des néovascularisations cornéennes, **caractérisé en ce qu'**il comprend le mélange d' un vecteur recombinant comprenant un acide nucléique codant pour un facteur anti-angiogénique avec un ou plusieurs adjuvants compatibles et pharmaceutiquement acceptables et l'imprégnation d'une lentille de contact.

9. Kit comprenant un récipient contenant une composition pharmaceutique comprenant un vecteur recombinant défectif comprenant au moins un acide nucléique codant pour un facteur anti-angiogénique et au moins une lentille de contact.

10. Kit selon la revendication 9, **caractérisé en ce que** la composition pharmaceutique comprend entre 1. 10⁶ et 1. 10¹² pfu / ml et de préférence entre 1. 10⁸ et 1. 10¹⁰ pfu / ml.

11. Utilisation du kit selon la revendication 9 ou 10 pour la préparation d'un médicament destiné à être administré par imprégnation d'une lentille pour la prévention, l'amélioration et/ou le traitement des néovascularisations cornéennes.

## Patentansprüche

1. Verwendung eines Vektors, der eine für einen antiangiogenen Faktor codierende Nukleinsäure umfasst, zur Herstellung einer pharmazeutischen Zusammensetzung, die dazu bestimmt ist, durch Imprägnierung einer weichen Linse und Aufbringen der Linse auf die Hornhaut zur Prävention, Verbesserung und/oder Behandlung von Hornhaut-Neovaskularisationen verabreicht zu werden.

2. Verwendung nach Anspruch 1, **dadurch gekennzeichnet, dass** die für den antiangiogenen Faktor codierende Nukleinsäure eine Nukleinsäure, die für ein Polypeptid codiert, ausgewählt aus dem N-terminalen Fragment des Plasminogenaktivators uPA (ATF), Angiostatin, Endostatin, dem 16 kDa-Fragment von Prolactin und dem Plättchenfaktor 4 (PF-4), oder eine Kombination von Nukleinsäuren ist, die für wenigstens zwei dieser Faktoren codiert.

3. Verwendung nach Anspruch 2, **dadurch gekennzeichnet, dass** der antiangiogene Faktor aus dem N-terminalen Fragment des Plasminogenaktivators uPA (ATF) und Angiostatin K3 ausgewählt ist.

4. Verwendung nach einem der Ansprüche 1 bis 3, **dadurch gekennzeichnet, dass** der Vektor ein Plasmid, ein Cosmid oder jede DNA, die nicht durch ein Virus eingekapselt ist, ist.

5. Verwendung nach einem der Ansprüche 1 bis 3, **dadurch gekennzeichnet, dass** der Vektor ein rekombinantes Virus ist, das vorzugsweise von einem Adenovirus, einem Retrovirus, einem Lentivirus, einem Herpes-Virus oder einem Adeno-assoziierten Virus abgeleitet ist.

6. Verwendung nach Anspruch 5, **dadurch gekennzeichnet, dass** das rekombinante Virus ein defektes rekombinantes Adenovirus ist.

7. Verwendung nach Anspruch 5 oder 6, **dadurch gekennzeichnet, dass** die pharmazeutische Zusammensetzung zwischen 1 · 10⁶ und 1 · 10¹² pfu/ml und vorzugsweise zwischen 1 · 10⁸ und 1 · 10¹⁰ pfu/ml umfasst.

8. Verfahren zur Herstellung eines Medikaments, das zur Prävention, Verbesserung und/oder Behandlung von Hornhaut-Neovaskularisationen nützlich ist, **dadurch gekennzeichnet, dass** es das Mischen eines rekombinanten Vektors, der eine für einen antiangiogenen Vektor codierende Nukleinsäure umfasst, mit einem oder mehreren Adjuvanzien, die kompatibel und pharmazeutisch annehmbar sind, und die Imprägnierung einer Kontaktlinse umfasst.

9. Kit, umfassend einen Behälter, der eine pharmazeutische Zusammensetzung, die einen defekten rekombinanten Vektor umfasst, welcher wenigstens eine für einen antiangiogenen Faktor codierende Nukleinsäure enthält, und wenigstens eine Kontaktlinse enthält.

10. Kit nach Anspruch 9, **dadurch gekennzeichnet, dass** die pharmazeutische Zusammensetzung zwischen 1 · 10⁶ und 1 · 10¹² pfu/ml und vorzugsweise zwischen 1 · 10⁸ und 1 · 10¹⁰ pfu/ml umfasst.

11. Verwendung des Kits nach Anspruch 9 oder 10 für die Herstellung eines Medikaments, das dazu bestimmt ist, durch Imprägnierung einer Linse zur Prävention, Verbesserung und/oder Behandlung von Hornhaut-Neovaskularisationen verabreicht zu werden.

## Claims

1. Use of a vector comprising a nucleic acid encoding an anti-angiogenic factor for the preparation of a pharmaceutical composition intended to be administered by impregnation of a soft lens and application of said lens on to the cornea, for the prevention, improvement and/or treatment of corneal neovascularization.

2. Use according to Claim 1, **characterized in that** the nucleic acid encoding the anti-angiogenic is a nucleic acid encoding a polypeptide chosen from the N-terminal fragment of the plasminogen activator uPA (ATF), angiostatin, endostatin, the 16 kDa fragment of prolactin or platelet factor 4 (PF-4) or a combination of nucleic acids encoding at least two of these factors.

3. Use according to Claim 2, **characterized in that** the anti-angiogenic factor is chosen from the N-terminal fragment of the plasminogen activator uPA (ATF) and angiostatin K3.

4. Use according to one of Claims 1 to 3, **characterized in that** the vector is a plasmid, a cosmid or any DNA not encapsidated by a virus.

5. Use according to one of Claims 1 to 3, **characterized in that** the vector is a recombinant virus, preferably derived from an adenovirus, a retrovirus, a lentivirus, a herpesvirus or an adeno-associated virus.

6. Use according to Claim 5, **characterized in that** the recombinant virus is a defective recombinant adenovirus.

7. Use according to Claim 5 or 6, **characterized in that** the pharmaceutical composition comprises between 1 × 10⁶ and 1 × 10¹² pfu/ml and preferably between 1 × 10⁸ and 1 × 10¹⁰ pfu/ml.

8. Process for preparing a medicinal product which is useful for the prevention, improvement and/or treatment of corneal neovascularization, **characterized in that** it comprises mixing a recombinant vector comprising a nucleic acid encoding an anti-angiogenic factor with one or more compatible and pharmaceutically acceptable adjuvants and impregnating a contact lens.

9. Kit comprising a recipient which contains a pharmaceutical composition comprising a defective recombinant vector comprising at least one nucleic acid encoding an anti-angiogenic factor and at least one contact lens.

10. Kit according to Claim 9, **characterized in that** the pharmaceutical composition comprises between 1 × 10⁶ and 1 × 10¹² pfu/ml and preferably between 1 × 10⁸ and 1 × 10¹⁰ pfu/ml.

11. Use of the kit according to Claim 9 or 10 for the preparation of a medicinal product intended to be administered via impregnation of a lens, for the prevention, improvement and/or treatment of corneal neovascularization.
